# EUROPEAN PATENT SPECIFICATION

(11) **EP 1 971 261 B1**
(45) Date of publication and mention of the grant of the patent: **30.10.2013**
(21) Application number: 07703744.8
(22) Date of filing: 09.01.2007
(51) Int. Cl.: A61B 5/0482

(54) **METHOD AND SYSTEM FOR REPRESENTING BRAIN ACTIVITIES**
VERFAHREN UND SYSTEM ZUR DARSTELLUNG VON HIRNAKTIVITÄTEN
MÉTHODE ET SYSTÈME DE REPRÉSENTATION D'ACTIVITÉS CÉRÉBRALES

(30) Priority: 10.01.2006 US 757784 P
(43) Date of publication of application: 24.09.2008
(73) Proprietor: Schoene, Martin, 31224 Peine (DE)
(72) Inventor: Schoene, Martin, 31224 Peine (DE)
(74) Representative: Patentanwälte Bressel und Partner
(86) International application number: PCT/EP2007/050197
(87) International publication number: WO 2007/080168

(56) References cited:
- WO-A-96/24906
- WO-A-2005/015523
- DE-U1- 20 309 484
- HENDRIK RASEHORN: "Schwingungen in der Petrischale" BRAUNSCHWEIGER ZEITUNG, [Online] 17 August 2004 (2004-08-17), XP002455178 Braunschweig Retrieved from the Internet: URL:http://realitaet.hbk-bs.de/new/pdf/bz_ artikel.pdf?FUIComponentClass=%5Btype+Func tion%5D&FScrollBarClass=%5Btype+Function%5 D&myXML=> [retrieved on 2007-10-15]
- HARALD LIKUS: "Ich hör' das Klopfen, ich seh' das Denken" BRAUNSCHWEIGER ZEITUNG, [Online] 4 February 2005 (2005-02-04), XP002455179 Braunschweig Retrieved from the Internet: URL:http://realitaet.hbk-bs.de/new/nachles e/presse/Presse%202005%20(Schulkowsky).pdf ?FUIComponentClass=%5Btype+Function%5D&FSc rollBarClass=%5Btype+Function%5D&myXML=> [retrieved on 2007-10-15]

## Description

The present invention relates generally, to the field of representation of brain activities and more specifically, to the representation of brain activities in the form of visual patterns.

The brain can have various states, for example, a calm state, an agitated state, a confused state, and an unconscious state. Further a brain activity can be associated with various parameters such as presence of a brain activity, absence of a brain activity and synchronization of different brain activities. Different combinations of a state of brain and brain activity parameter are possible such as relaxed state with synchronization, synchronization with activity and so forth.

Several techniques are known in the art for the representation of brain activities. A brain activity may be represented in the form of a signal. Examples of these signals include electrical signals. Existing methods that represent brain activities include Electroencephalogram (EEG). An EEG is recorded by connecting electrodes to a subject's scalp. These electrodes pick up electrical signals that are naturally produced by the brain and transmit them to galvanometers present in an EEG recorder. These galvanometers are connected to pens to trace the electrical signal onto a continuously moving graph paper.

However, the existing systems suffer from following limitations. The signals on the EEG may not be interpreted by a layman. Further, since the EEG is represented in the form of a graph, it may be difficult and time-consuming to compare different signals. Also, one cannot recognize synchronization of brain activities through EEG, without computations.

In light of the foregoing discussion, there is a need for representing brain activities in the form of user friendly signals. The output signal should be such that the user can easily interpret it. Further, there is a need for representing brain activities such that the user is able to visualize a change in the brain activity through the corresponding output signal. The representation should also enable easy comparison of different brain activities.

The article 'Schwingungen in der Petrischale', Hendrick Rosehorn, Braunschweiger Zeitung, page 20, 17.08.2004, discloses a representation of brain activity in the form of water patterns.

An object of the invention is to represent brain activities in the form of visual patterns.

Another object of the invention is to represent brain activities in a form that is easy to interpret by a layman.

Another object of the invention is to represent brain activities such that a user is able to visualize a change in the brain activity through the corresponding output signal.

The invention provides a system for representing brain activities in the form of visual patterns. The system comprises a signal frequency modulator (frequenzer) and a resonating chamber. The signal frequenzer receives signals corresponding to a brain activity. The signal frequenzer then generates an audio signal and modulates the audio signal using the signal corresponding to the brain activity. The resonating chamber generates a visual pattern corresponding to the audio signal from the signal frequenzer: The resonating chamber comprises a loudspeaker, a bowl, and a cold-light electrode. The loudspeaker comprises a magnet, two siphons and a diaphragm. The magnet vibrates after receiving the output signal from the signal frequenzer. The magnet induces the diaphragm to vibrate. The diaphragm in turn results in the vibration of the bowl. The bowl contains a liquid that vibrates along with the bowl and a state of resonance is achieved. The light emitted by the cold-light electrode is incident on the vibrating liquid in the bowl, resulting in the generation of a visual pattern at resonance. In an embodiment of the invention, a camera records the visual pattern. In yet another embodiment of the invention, a display device displays the recorded visual pattern.

The invention further provides a portable system for representing brain activities in the form of visual patterns. The portable system comprises a signal frequenzer and a resonating chamber. The signal frequenzer receives a signal corresponding to a brain activity. The signal frequenzer generates an audio signal and modulates the audio signal by the signal corresponding to the brain activity. The resonating chamber comprises a magnetic coil, a bowl and a cold-light electrode placed above the bowl. The magnetic coil vibrates on receiving the output signal from the signal frequenzer. The bowl, which is in contact with the magnetic coil, vibrates along with the magnetic coil. The bowl contains liquid that vibrates along with the bowl. The light emitted by the cold-light electrode placed above the bowl is incident on the vibrating liquid, generating a visual pattern. The portable system further comprises a camera that records the visual pattern.

The invention also provides a method for representing brain activities in the form of visual patterns. The method comprises the steps of receiving a signal corresponding to a brain activity; generating an audio signal corresponding to the received signal; and generating a visual pattern corresponding to the audio signal. In an embodiment of the invention, the method further comprises recording the visual pattern. In an embodiment of the invention, the method further comprises displaying the recorded visual pattern.

The various embodiments of the invention will hereinafter be described in conjunction with the appended drawings, provided to illustrate and not to limit the invention, wherein like designations denote like elements, and in which:
FIG. 1 is a block diagram of a system for representing brain activities, in accordance with an embodiment of the invention.
FIGs. 2A and 2B illustrate a resonating chamber, in accordance with an embodiment of the invention.
FIGs. 3A and 3B illustrate a light box, in accordance with an embodiment of the invention.
FIG. 4 illustrates an integrated view of resonating chamber, camera and light box, in accordance with an embodiment of the invention.
FIGs. 5A and 5B illustrate various views of the resonating chamber, in accordance with an embodiment of the invention.
FIGs. 6A and 6B illustrate various views of the light box, in accordance with an embodiment of the invention.
FIG. 7 illustrates a portable system for representing brain activities, in accordance with an embodiment of the invention.
FIG. 8 is a flowchart of a method for representing brain activities, in accordance with an embodiment of the invention.
FIGs. 9A and 9B, are visual patterns, in accordance with an exemplary embodiment of the invention.

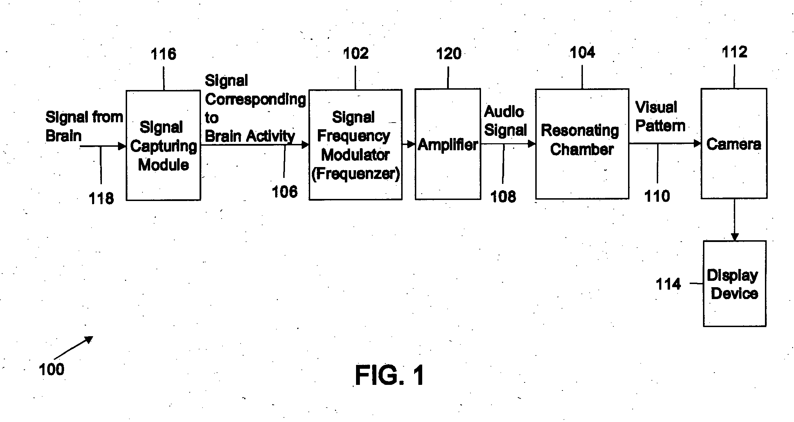

FIG. 1 is a block diagram of a system 100 for representing brain activities, in accordance with an embodiment of the invention.

System 100 comprises a signal frequency modulator (frequenzer) 102 and a resonating chamber 104. Signal frequenzer 102 receives a signal 106. Signal 106 is a signal corresponding to a brain activity. In an embodiment of the invention, signal 106 is an electrical signal. Signal frequenzer 102 generates an audio signal, which is modulated by signal 106, to generate audio signal 108. In an embodiment of the invention, audio signal 108 has a frequency ranging from 1 cycle per second to 1000 cycles per second.

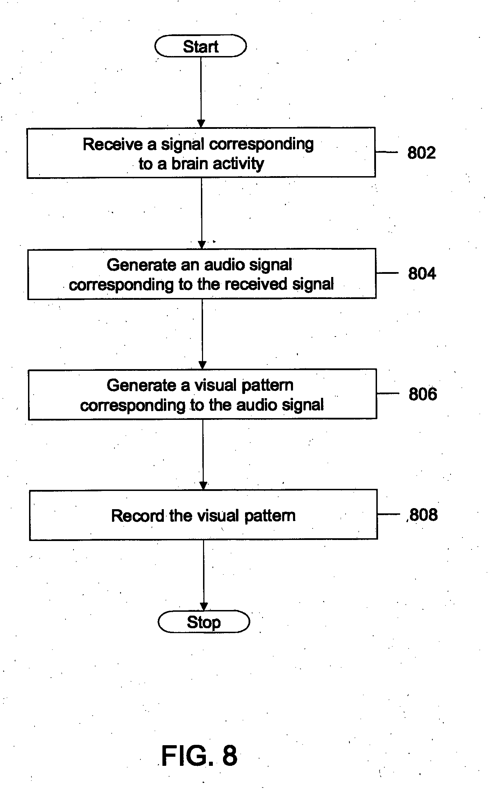

Resonating chamber 104 generates a visual pattern 110 corresponding to audio signal 108. The generation of visual pattern is further explained in conjunction with FIG. 8. In an embodiment of the invention, system 100 comprises an amplifier 120 that amplifies audio signal 108.

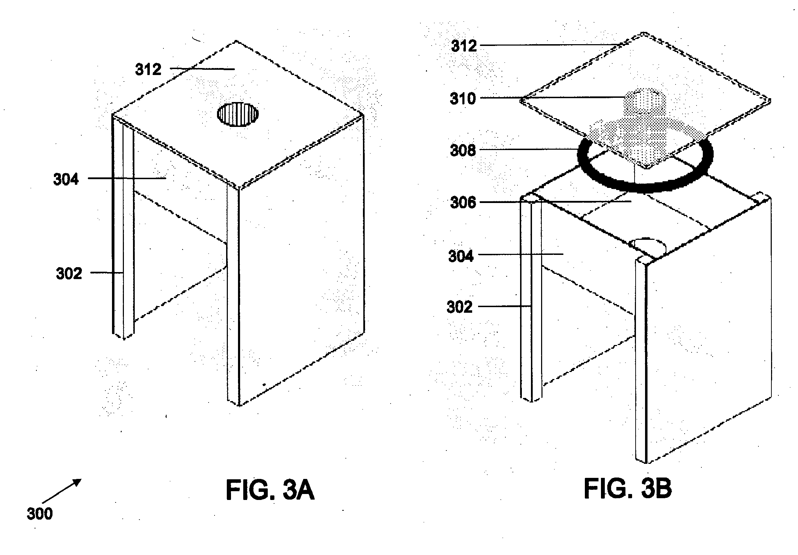

In an embodiment of the invention, system 100 further includes a camera 112. Camera 112 records visual pattern 110. In an embodiment of the invention, camera 112 is a high-resolution camera. Examples of camera 112 include a digital camera, a hard- disc-recording camera and the like. In an embodiment of the invention, system 100 further includes a display device 114. Display device 114 displays visual pattern 110. Examples of display device 114 include television screen and a beamer. In an embodiment of the invention, camera 112 is placed in a light box to improve the quality of visual pattern 110. The quality of visual pattern 110 is measured in terms of parameters such as brightness and resolution of visual pattern 110. The light box is further explained in conjunction with FIG. 3.

In an embodiment of the invention, system 100 further includes a signal-capturing module 116. Signal-capturing module 116 captures a signals 118, corresponding to a brain activity and generates signal 106, corresponding to signal 118. In an embodiment of the invention, signal-capturing module 116 is an Electroencephalograph for recording an Electroencephalogram (EEG) 106. For example, signal-capturing module 116 captures signals from the brain, corresponding to a calm state of the brain and provides signal 106, in an electrical form, corresponding to the calm state of brain.

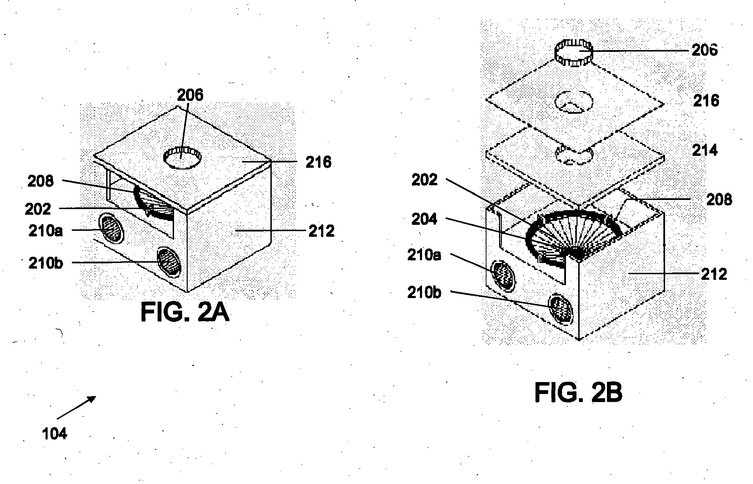

FIGs. 2A and 2B illustrate resonating chamber 104, in accordance with an embodiment of the invention. Resonating chamber 104 includes a loudspeaker. Loudspeaker comprises a diaphragm 202 attached to a central magnet 204. For example, diaphragm 202 may be a sheet of a film or foil, which vibrates in response to audio signal 108. Central magnet 204 induces diaphragm 202 to initiate vibration. In an embodiment of the invention, diaphragm 202 is a low-frequency diaphragm. In an embodiment of the invention, diaphragm 202 has a frequency range >from 500 to 3000 hertz (Hz). Resonating chamber further comprises a bowl 206, and a first cold-light-electrode 208. Bowl 206 vibrates in response to the vibrations of diaphragm 202. Bowl 206 holds a liquid that vibrates in response to the vibrations of bowl 206. For example, bowl 206 may be filled with a liquid such as water. Bowl 206 is made of a transparent material. For example, bowl 206 may be made of glass. The vibrating diaphragm, vibrating bowl and vibrating liquid achieve a state of resonance. First cold-light-electrode 208 emits light. In an embodiment of the invention, first cold-light-electrode 208 emits light such that there is no emission of heat with the light. In an embodiment of the invention, first cold-light-electrode 208 is a fluorescent lamp. The fluorescent lamp may have various shapes and sizes. In another embodiment of the invention, first cold-light-electrode 208 is a neon-lamp. In yet another embodiment of the invention, first cold-light-electrode 208 is a Light Emitting Diode (LED), which is placed between diaphragm 202 and bowl 206, as shown in FIGs. 2A and 2B. First cold-light electrode 208 emits light that is incident on vibrating liquid in bowl 206. The light falling on the vibrating liquid generates a pattern. The pattern of vibrating liquid forms visual pattern11.0.

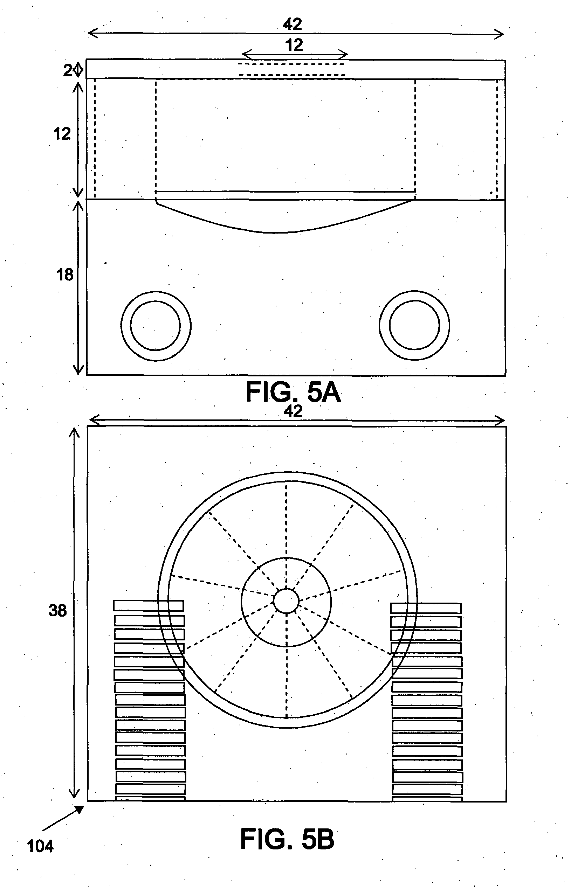

In an embodiment of the invention, the loudspeaker further includes a plurality of siphons, for example siphon 210a and siphon 210b. Siphon 210a and siphon 210b allow the air to enter into resonating chamber 104 such that the air lifts diaphragm 202. Diaphragm 202, bowl 206, first cold-light-electrode 208, siphon 210a and siphon 210b are placed in a box 212, as shown in FIGS. 2A and 2B. In an embodiment of the invention, resonating chamber 104 further includes a styrofoam plate 214. Styrofoam plate 214 has a hole at the centre. The shape and size of the hole is so designed that the base of bowl 206 fits into the hole of styrofoam plate 214. This provides a base for bowl 206. In an embodiment of the invention, resonating chamber 104 further includes a black fabric plate 216. Black fabric plate 216 is a non-reflective plate and contains the light emitted by first cold-light-electrode 208 within resonating chamber 104. The non-reflective property of black fabric plate 216 results in blocking light from any external source to interfere with visual pattern 110. An exemplary embodiment of resonating chamber 104 is further explained in conjunction with FIG. 5.

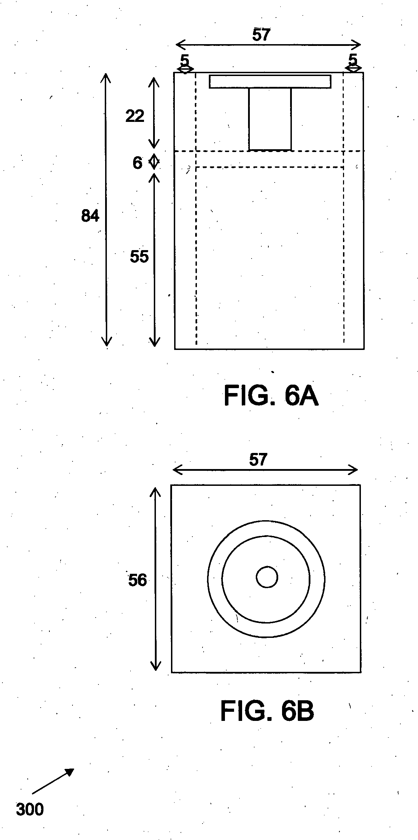

FIG. 3 illustrates light box 300, in accordance with an embodiment of the invention. As shown in FIG. 3, the assembly of light box 300 is supported on four wooden construction bars 302 and four side-panels 304. Light box 300 includes a diffusor plate 306, a second cold-light electrode 308 and a tube 310. Diffusor plate 306 guides light emitted by second cold-light electrode 308 in such a manner that the light is evenly incident on the liquid in bowl 206 and the recording of visual pattern 110 is bright. In an embodiment of the invention, second cold-light electrode 308 is a neon-lamp. In another embodiment of the invention, second cold-light electrode 308 is a LED. For example, second cold-light electrode 308 may be a 20 x 1 Watt LED (20 LEDs of 1 Watt each). Tube 310 transfers visual pattern 110 from resonating chamber 104 to camera 112. In an embodiment of the invention, tube 310 is made of aluminum. In an embodiment of the invention, light box 300 further includes a cover plate 312. Cover plate 312 blocks light >from any external source to interfere with the recording by camera 112. Cover plate 312 has a hole at the centre. The shape and size of the hole are so designed that the lens of camera 112 fits in the hole. An exemplary embodiment of light box 300 is further explained in conjunction with FIG. 6.

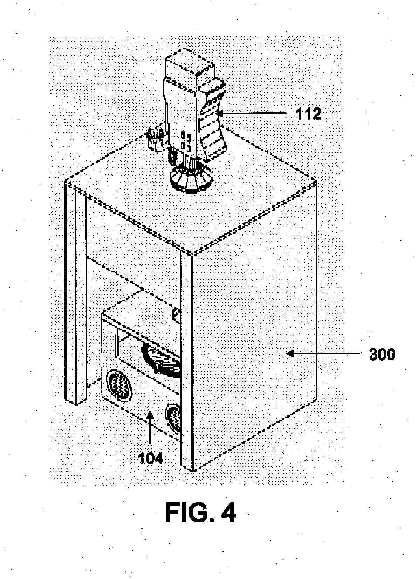

FIG. 4 illustrates an integrated view of resonating chamber 104, camera 112 and light box 300, in accordance with an embodiment of the invention. As shown in FIG. 4, light box 300 is placed above resonating chamber 104 and camera 112 is so placed in light box 300 that the lens of camera 112 fits into the hole of cover plate 312. Camera 112 is aligned with tube 310, which is further aligned with bowl 206.

FIGs. 5A and 5B illustrate various views of a resonating chamber 104, in accordance with an embodiment of the invention. FIG. 5A is a front view of resonating chamber 104. FIG. 5B is a top view of resonating chamber 104. As shown in FIGs. 5A and 5B, resonating chamber 104 has a height of 32 centimeters (cm); length of 42 cm and width of 38 cm. Bowl 206 is placed at the centre along the length of resonating chamber 104, which has a diameter of 12 cm. The loudspeaker is placed at a height of 18 cm. The thickness of styrofoam plate 214 is 2 cm. Diaphragm 202 has a diameter of 33 cm. Black fabric plate 216 has a thickness ranging from 19 to 25 mm. First cold-light-electrode 208 is a fluorescent lamp. Fluorescent lamp has a circular shape with a diameter of 380 millimeters (mm).

FIGs. 6A and 6B illustrate the various views of light box 300, in accordance with an embodiment of the invention. FIG. 6A is a front view of light box 300. FIG. 6B is a top view of light box 300. As shown in FIGs. 6A and 6B, light box 300 has a height of 84 centimeters (cm), length of 57 cm and width of 56 cm. Light box 300 has two construction bars each, with a width of 5 cm. The bottom portion of light box 300 with dimensions 47 cm x 56 cm x 55 cm is hollow. Side-panel 304 is at a height of 55 cm. Tube 310 is a hollow, cylindrical and placed at the centre along the length of light box 300. Tube 310 has a diameter of 13 cm and a height of 22 cm. Further, second cold-light electrode 308 is circular in shape and has a diameter in the range of 40 to 45 cm. Second cold-light electrode 308 is placed 1 cm below the top-most portion of light box 300.

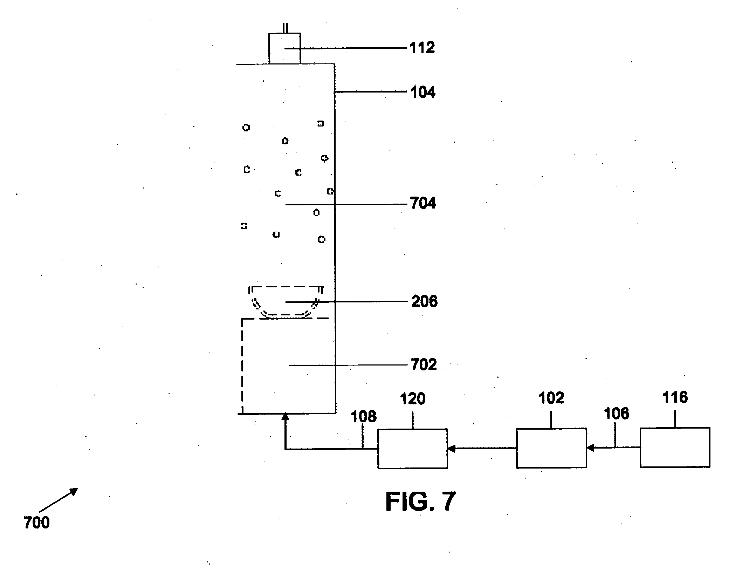

FIG. 7 illustrates a portable system 700 for representing brain activities, in accordance with an embodiment of the invention. Portable system 700 comprises signal- frequenzer 102, and resonating chamber 104. In an embodiment of the invention, resonating chamber 104 is cylindrical in shape. Signal frequenzer receives signal 106 corresponding to the brain activity. Signal frequenzer 102 generates an audio signal and modulates the audio signal using signal 106, to generate audio signal 108. Resonating chamber 104 comprises a magnetic coil 702, a bowl 206, a third cold-light electrode 704. Bowl 206 is placed on magnetic coil 702. Third cold-light electrode 704 is placed above bowl 206. Magnetic coil 702 vibrates in response to audio signal 108. Bowl 206 vibrates along with magnetic coil 702. Bowl 206 is filled with a liquid. For example, bowl 206 may be filled with water. The liquid present in bowl 206 vibrates along with bowl 206. The vibrating magnetic coil 702, vibrating bowl 206 and vibrating liquid achieve a state of resonance. The light, emitted by third cold-light electrode 704, incident on the vibrating liquid generates visual pattern 110.

In an embodiment of the invention, portable system 700 comprises a signal-capturing module 116. In an embodiment of the invention, portable system 700 comprises a camera 112, which captures visual pattern 110. In an embodiment of the invention, portable system 700 comprises an amplifier 120 that amplifies audio signal 108.

In an exemplary embodiment of portable system 700, resonating chamber is cylindrical in shape. The diameter of the cylinder is 16 cm. The length of the cylinder is 55 cm. The cylinder is made of black Plexiglas material with a lining of mirror-foil inside. The length of magnetic coil 702 is 15 cm. The diameter of bowl 206 is 12 cm and the height of bowl 206 is 5 cm. Third cold-light electrode 704 is a plurality of LEDs. For example, there may be 12 white LEDs of 1 Watt each. Camera 112 is a digital video camera.

FIG. 8 is a flowchart of a method for representing brain activities, in accordance with an embodiment of the invention. At step 802, signal 106, corresponding to a brain activity is received. Signal 106 is received by signal frequenzer 102. In an embodiment of the invention, signal frequenzer 102 receives signal 106 through signal-capturing module 116. Signal-capturing module 116 obtains signal 118 directly from the brain and provides a corresponding signal 106. In an embodiment of the invention, signal-capturing module 116 is an electroencephalograph and signal 106 is an EEG.

At step 804, audio signal 108 corresponding to signal 106 is generated. An audio signal generated by signal frequenzer 102 is modulated using signal 106 to generate audio signal 108. At step 806, visual pattern 110 corresponding to audio signal 108 is generated. Visual pattern 110 is generated by resonating chamber 104. The functioning of resonating chamber 104 is described in conjunction with FIGS. 1, 2 and 7. In an embodiment of the invention, at step 808, visual pattern 110 may be recorded by using camera 112. Further, the recorded visual pattern 110 may be displayed on display device 114 such as a television and a beamer.

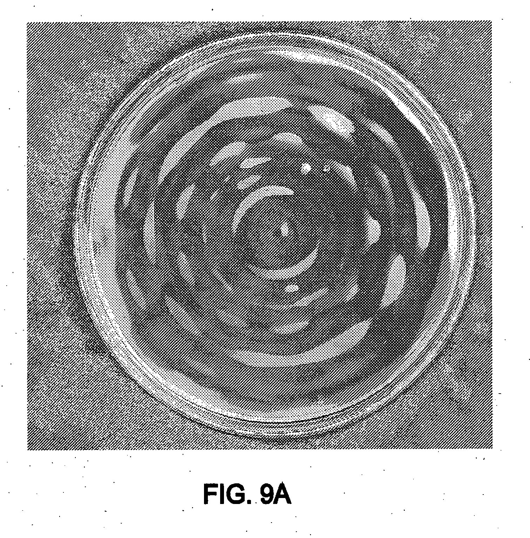

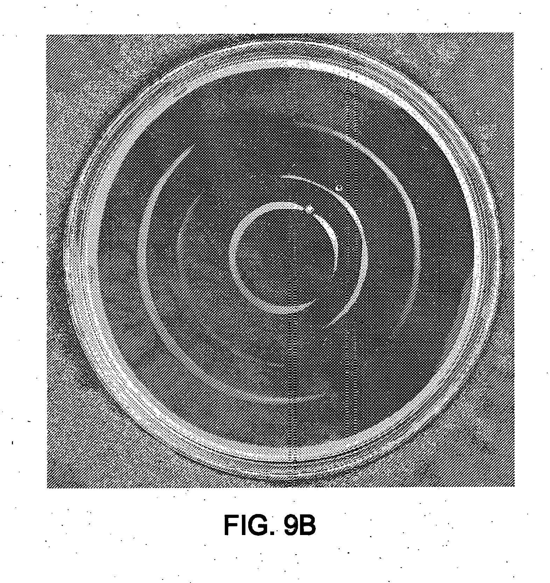

FIGs. 9A and 9B illustrate an exemplary visual pattern. FIG. 9A is a visual pattern that represents activity of the brain when the brain is in a relaxed state and there is no synchronization between the brain activities. No synchronization between the brain activities implies that thinking, feeling and noticing are not forming a unit and they are not working together. For example, the brain of a person who is drinking a cup of coffee, and simultaneously thinking about the taste of the coffee and about getting late for a meeting may produce a visual pattern similar to the one shown in FIG. 9A.

FIG. 9B is a diagram of a visual pattern that represents activity of the brain when the brain is in a relaxed state and there is synchronization between the brain activities. Synchronization between the brain activities implies that the brain is in the condition of an optimal information flow and that all information from the brain is co-operating in a harmonious condition of internal peace. For example, the brain of a person who is drinking a cup of coffee and thinking about only coffee and has no other thoughts, may produce a visual pattern similar to the one shown in FIG. 9B.

In various embodiments of the invention, the system may be used as a feedback instrument by a layman. The visual pattern provided by the system corresponding to the brain activities may be easily interpreted by a layman. Further, the layman may confirm a particular state of brain using the information interpreted through a visual pattern. Further, in various embodiments of the invention, the user can know the synchronization state of his activities and evaluate his or her concentration levels.

While the preferred embodiments of the invention have been illustrated and described, it will be clear that the invention is not limited to these embodiments only. Numerous modifications, changes, variations, substitutions and equivalents will be apparent to those skilled in the art without departing from the scope of the invention as described in the claims.

## Claims

1. A system (100) for representing brain activities in the form of visual patterns, the system (100) comprising:
a. a signal frequency modulator (102) for receiving a signal (118) corresponding to a brain activity and generating an audio signal (108) corresponding to the received signal (118); and
b. a resonating chamber (104) for generating a visual pattern corresponding to the audio signal (108); wherein the resonating chamber (104) comprises:
a loudspeaker, the loudspeaker comprising:
a diaphragm (202); and
a magnet (204), the magnet (204) configured to induce the diaphragm (202) to vibrate
in response to the audio signal (108);
a bowl (206), the bowl (206) being filled with a liquid;
**characterized in that**
the resonanting chamber (104) comprises
a first cold-light-electrode, the first cold-light-etectrode configured to emit light,
wherein the first cold-light-electrode is placed between the diaphragm (202) and the bowl (206).

2. The system of claim 1 further comprising a signal capturing module (118), the signal capturing module (116) capturing the signal (118) corresponding to the brain activity.

3. The system of claim 2, wherein the signal capturing module is an Etectroencephalogram (EEG).

4. The system of claim 1, wherein the loudspeaker comprises one or more siphons (210a, 210b), the one or more siphons (210a, 210b) forcing air through the resonating chamber (104) at atmospheric pressure.

5. The system of claim 1, the resonating chamber further comprises a styrofoam plate (214), the styrofoam plate (214) providing a base for the bowl (206).

6. The system of claim 1, wherein the resonating chamber (104) further comprises a black fabric plate (216), the black fabric plate (216) containing the light within the resonating chamber (104).

7. The system of claim 1, wherein the first cold-light-electrode is a fluorescent lamp.

8. The system of claim 1 further comprising a camera (112), the camera (112) recording the visual pattern.

9. The system of claim 8 further comprising a light box (300), wherein the camera (112) is placed in the light box.

10. The system of claim 9, wherein the light box (300) comprises:
a a diffusor plate (306);
b a second cold-light electrode (308), the second cold-light-electrode emitting light; and
c a tube (310), the tube (310) transferring the visual pattern from the bowl to the camera (112).

11. The system of claim 10, wherein the light (300) box further comprises a cover plate.

12. The system of claim 8 further comprises a display device, the display device displaying the recorded visual pattern.

13. The system of claim 1 further comprising an amplifier, the amplifier amplifying the audio signal (10B).

14. A method for representing brain activities in the form of visual patterns, the method comprising:
a. receiving (102) a signal corresponding to a brain activity;
b. generating (120) an audio signal (108) corresponding to the received signal; and
c. generating a visual pattern corresponding to the audio signal by a resonating chamber (104) comprising:
a loudspeaker, the loudspeaker comprising
a diaphragm (202); and
a magnet (204), the magnet (204) configured to induce the diaphragm (202) to vibrate in response to the audio signal (108);
a bowl (206), the bowl (206) being filled with a liquid;
**characterized in that**
the resonanting chamber (104) comprises
a first cold-light-electrode, the first cold-light-electrode emitting light,
wherein the first cold-light-electrode is placed between the diaphragm (202) and the bowl (206).

15. The method of claim 14 further comprising recording the visual pattern,

16. The method of claim 15 further comprising displaying the recorded visual pattern.

## Patentansprüche

1. System (100) zur Darstellung von Gehirnaktivität in Form visueller Muster, wobei das System (100), umfasst:
a. einen Frequenz-Modulator (102) zum Empfangen eines Signals (118) entsprechend der Aktivität des Gehirns und zum Erzeugen eines Audio-Signals (108) entsprechend dem empfangenen Signal (118), und
b. eine Resonanzkammer (104) zum Erzeugen eines visuellen Musters entsprechend dem Audiosignal (108), wobei die Resonanzkammer (104) umfasst:
einen Lautsprecher, wobei der Lautsprecher umfasst:
eine Membran (202);
und einen Magneten (204), wobei der Magnet (204) derart konfiguriert ist, dass die Membran (202) angeregt wird und als Reaktion auf das Audio-Signal (108) schwingt; eine Schale (206), wobei die Schale (206) mit einer Flüssigkeit gefüllt ist;
**dadurch gekennzeichnet, dass** die Resonanzkammer (104) eine erste Kaltlicht-Elektrode umfasst, wobei die erste Kaltlicht-Elektrode konfiguriert ist, Licht zu emittieren, wobei die erste Kaltlicht-Elektrode zwischen der Membran (202) und der Schale (206) angeordnet ist.

2. Das System nach Anspruch 1 umfassend ferner ein Signalerfassungs-Modul (116), wobei das Signalerfassungs-Modul (116) das Signal (118) entsprechend der Aktivität des Gehirns erfasst.

3. Das System nach Anspruch 2, wobei das Signalerfassungs-Modul ein Elektroenzephalogramm (EEG) ist.

4. Das System nach Anspruch 1, wobei der Lautsprecher ein oder mehrere Luftkanäle (210a, 210b) umfasst, wobei der ein oder die mehreren Luftkanäle (210a, 21 0b) Luft durch die Resonanzkammer (104) bei atmosphärischem Druck treiben.

5. Das System nach Anspruch 1, wobei die Resonanzkammer (104) ferner eine Styropor-Platte (214) umfasst, wobei die Styropor-Platte (214) eine Basis für die Schale (206) bildet.

6. Das System nach Anspruch 1, wobei die Resonanzkammer (104) ferner eine schwarze Stoff-Platte (216) umfasst, wobei die schwarze Stoff-Platte (216) das Licht innerhalb der Resonanzkammer (104) hält.

7. Das System nach Anspruch 1, wobei die erste Kaltlicht-Elektrode eine Leuchtstofflampe ist.

8. Das System nach Anspruch 1 umfassend weiterhin eine Kamera (112), wobei die Kamera (112) das visuelle Muster aufzeichnet.

9. Das System nach Anspruch 8 umfassend ferner einen Lichtkasten (300), wobei die Kamera (112) in dem Lichtkasten platziert ist.

10. Das System nach Anspruch 9, wobei der Lichtkasten (300) ferner umfasst:
a. eine Diffusor-Platte (306);
b. eine zweite Kaltlicht-Elektrode (308), wobei die zweite Kaltlicht-Elektrode Licht emittiert, und
c. ein Rohr (310), wobei das Rohr (310) das optische Muster aus der Schale zu der Kamera (112) überträgt.

11. Das System nach Anspruch 10, wobei der Lichtkasten (300) ferner eine Abdeckplatte umfasst.

12. Das System nach Anspruch 8 umfassend ferner eine Anzeigevorrichtung, wobei die Anzeigevorrichtung die aufgenommenen visuellen Muster darstellt.

13. Das System nach Anspruch 1 umfassend ferner einen Verstärker, wobei der Verstärker das Audio-Signal (108) verstärkt.

14. Ein Verfahren zur Darstellung von Gehirnaktivität in Form von visuellen Mustern, wobei das Verfahren umfasst:
a. Empfangen (102) eines Signals entsprechend einer Aktivität des Gehirns;
b. Erzeugen (120) eines Audiosignals (108) entsprechend dem empfangenen Signal; und
c. Erzeugen eines optischen Musters entsprechend dem Audiosignal durch eine Resonanzkammer (104) umfassend:
einen Lautsprecher; wobei der Lautsprecher umfasst
eine Membran (202), und
einen Magneten (204), wobei der Magnet (204) konfiguriert ist, um die Membran (202) zu veranlassen, in Reaktion auf das Audio-Signal (108) zu schwingen;
eine Schale (206); wobei die Schale (206) mit einer Flüssigkeit gefüllt ist;
**dadurch gekennzeichnet, dass** die Resonanzkammer (104) eine erste Kaltlicht-Elektrode umfasst, wobei die erste Kaltlicht-Elektrode Licht emittiert,
wobei die erste Kaltlicht-Elektrode zwischen der Membran (202) und der Schale (206) angeordnet ist.

15. Die Methode nach Anspruch 14 umfassend ferner das Aufzeichnen des visuellen Musters.

16. Die Methode nach Anspruch 15 umfassend ferner das Anzeigen des aufgezeichneten visuellen Musters.

## Revendications

1. Système (100) représentant l'activité cérébrale sous forme de modélisations visuelles; le système (100) comprend:
a. un modulateur de fréquence (102) recevant un signal (118) correspondant à l'activité cérébrale et générant un signal audio (108) correspondant au signal reçu (118); et
b. une chambre de résonance (104) générant une modélisation visuelle correspondante au signal audio; la chambre de résonance (104) comprend:
un haut-parleur; le haut-parleur comprend:
une membrane (202), et
un aimant (204); l'aimant (204) est configuré pour faire vibrer la membrane en réponse au signal audio (108);
une cuvette (206); la cuvette (206) est remplie par un liquide en ce sens que la chambre de résonance (104) comprenne une première électrode à lumière froide; la première électrode à lumière froide est configurée pour émettre de la lumière. La première électrode à lumière froide est placée entre la membrane (202) et la cuvette (206).

2. Le système énoncé au point 1 comprend en outre un module de détection de signal (116); le module de détection de signal (116) détecte le signal (118) correspondant à l'activité cérébrale.

3. Le module de détection de signal énoncé au point 2 est un électroencéphalogramme (EEG).

4. Dans le système énoncé au point 1, le haut-parleur comprend un ou plusieurs siphons (210a, 210b); le ou les siphons (21 0a, 210b) forcent l'air à passer à travers la chambre de résonance (104) grâce à la pression atmosphérique.

5. Dans le système énoncé au point 1, la chambre de résonance comprend en outre une plaque de polystyrène (214); la plaque de polystyrène (214) fournit une base pour la cuvette (206).

6. Dans le système énoncé au point 1, la chambre de résonance (104) comprend en outre une plaque de tissu noir (216); la plaque de tissu noir (216) contient la lumière à l'intérieur de la chambre de résonance (104).

7. Dans le système énoncé au point 1, l'électrode à lumière froide est une lampe fluorescente.

8. Le système énoncé au point 1 comprend en outre une caméra (112); la caméra (112) enregistre la modélisation visuelle.

9. Le système énoncé au point 8 comprend en outre un caisson lumineux (300); la caméra (112) est placée dans le caisson lumineux.

10. Dans le système énoncé au point 9, le caisson lumineux comprend:
a. une plaque de diffusion (306);
b. une seconde électrode à lumière froide (308); la seconde électrode à lumière froide émet de la lumière;
c. un tuyau (310); le tuyau (310) transfert la modélisation visuelle de la cuvette à la caméra (112).

11. Dans le système énoncé au point 10, le caisson lumineux comprend en outre une plaque de couverture.

12. Le système énoncé au point 8 comprend en outre un dispositif d'affichage; le dispositif d'affichage affiche la modélisation visuelle enregistrée.

13. Le système énoncé au point 1 comprend en outre un amplificateur; l'amplificateur amplifie le signal audio (108).

14. Une méthode de représentation de l'activité cérébrale sous forme de modélisation visuelle; la méthode comprend:
a. la réception (102) d'un signal correspondant à l'activité cérébrale;
b. la génération (120) d'un signal audio(108) correspondant au signal reçu;
c. la génération d'une modélisation visuelle correspondante au signal audio grâce à une chambre de résonance (104) comprenant:
un haut-parleur; le haut parleur comprenant
une membrane (202), et
un aimant (204); l'aimant (204) est configuré pour faire vibrer la membrane en réponse au signal audio (108);
une cuvette (206); la cuvette (206) est remplie par un liquide en ce sens que la chambre de résonance (104) comprennne une première électrode à lumière froide; la première électrode à lumière froide émet de la lumière. La première électrode à lumière froide est placée entre la membrane (202) et la cuvette (206).

15. La méthode énoncée au point 14 comprend en outre l'enregistrement des modélisations visuelles.

16. La méthode énoncée au point 15 comprend en outre l'affichage des modélisations visuelles enregistrées.
